# EUROPEAN PATENT SPECIFICATION

(11) **EP 4 529 478 B1**
(45) Date of publication and mention of the grant of the patent: **14.01.2026**
(21) Application number: 23728864.2
(22) Date of filing: 04.05.2023
(51) Int. Cl.: A61M 1/00

(54) **AN INDEPENDENT MACROSTRAIN DEVICE**
UNABHÄNGIGE MAKROSPANNUNGSVORRICHTUNG
DISPOSITIF DE MACROTENSION INDÉPENDANT

(30) Priority: 23.05.2022 US 202263344737 P
(43) Date of publication of application: 02.04.2025
(73) Proprietor: Solventum Intellectual Properties Company, Maplewood, MN 55144 (US)
(72) Inventor: MCGREGOR, Andrew J., Saint Paul, Minnesota 55133-3427 (US); KAZALA, Richard M., Jr., Saint Paul, Minnesota 55133-3427 (US)
(74) Representative: Simmons & Simmons
(86) International application number: PCT/IB2023/054645
(87) International publication number: WO 2023/227973

(56) References cited:
- US-A1- 2018 256 406

## Description

### TECHNICAL FIELD

The disclosure herein relates generally to tissue treatment systems and more particularly to dressing interfaces for use with tissue treatment systems

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

There is also widespread acceptance that cleansing a tissue site can be highly beneficial for new tissue growth. For example, a wound or a cavity can be washed out with a liquid solution for therapeutic purposes. These practices are commonly referred to as "irrigation" and "lavage" respectively. "Instillation" is another practice that generally refers to a process of slowly introducing fluid to a tissue site and leaving the fluid for a prescribed period of time before removing the fluid. For example, instillation of topical treatment solutions over a wound bed can be combined with negative-pressure therapy to further promote wound healing by loosening soluble contaminants in a wound bed and removing infectious material. As a result, soluble bacterial burden can be decreased, contaminants removed, and the wound cleansed.

While the clinical benefits of negative-pressure therapy are widely known, improvements to therapy systems, components, and processes may benefit healthcare providers and patients.
US2018/0256406 discloses an abdominal treatment system for providing reduced-pressure treatment to an abdominal cavity, a deep-tissue closure device for applying a closing force on a deep-tissue wound; and a surface-wound closure subsystem for providing a closing force on a surface wound

### BRIEF SUMMARY

The invention is defined by the independent claim. A selection of optional features is set out in the dependent claims.

### BRIEF DESCRIPTION OF THE DRAWINGS

Figure 1 is a functional block diagram of an example embodiment of a therapy system that can provide negative-pressure treatment and instillation treatment in accordance with this specification;
Figure 2 is a top view of a dressing interface that may be associated with some embodiments of the therapy system of Figure 1;
Figure 3 is a bottom view of the dressing interface of Figure 2, illustrating additional details that may be associated with some embodiments;
Figure 4 is a cross-section view of the dressing interface of Figure 2, illustrating additional details that may be associated with some embodiments;
Figure 5 is a top view of another example embodiment of a dressing interface that may be associated with some embodiments of the therapy system of Figure 1;
Figure 6A is a cross-section of the dressing interface of Figure 5, illustrating additional details that may be associated with some embodiments;
Figure 6B is a cross-section of the dressing interface of Figure 5 under negative pressure, illustrating additional details that may be associated with some embodiments;
Figure 6C is a cross-section of the dressing interface of Figure 5, illustrating additional details that may be associated with some embodiments;
Figure 7 is a top view of another example embodiment of the dressing interface of Figure 5, illustrating additional details that may be associated with some embodiments;
Figure 8 is a top view of another example embodiment of a dressing interface that may be associated with some embodiments of the therapy system of Figure 1; and
Figure 9 is a top view of the dressing interface of Figure 8, illustrating additional details that may be associated with some embodiments.

### DESCRIPTION OF EXAMPLE EMBODIMENTS

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

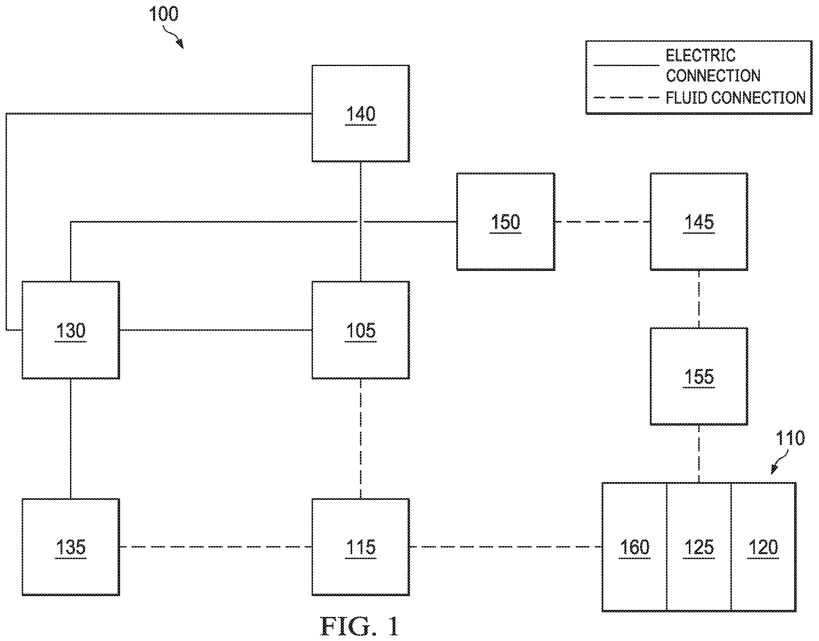

Figure 1 is a simplified functional block diagram of an example embodiment of a therapy system 100 that can provide negative-pressure therapy with instillation of topical treatment solutions to a tissue site in accordance with this specification.

The term "tissue site" in this context broadly refers to a wound, defect, or other treatment target located on or within tissue, including, but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness bums, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a source or supply of negative pressure, such as a negative-pressure source 105, and one or more distribution components. A distribution component is preferably detachable and may be disposable, reusable, or recyclable. A dressing, such as a dressing 110, and a fluid container, such as a container 115, are examples of distribution components that may be associated with some examples of the therapy system 100. As illustrated in the example of Figure 1, the dressing 110 may comprise or consist essentially of a tissue interface 120, a cover 125, or both in some embodiments. The dressing 110 may also include a macrostrain device, such as a dressing interface 160, in some embodiments.

A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include or comprise interfaces or fluid ports to facilitate coupling and de-coupling other components. In some embodiments, for example, a dressing interface may facilitate coupling a fluid conductor to the dressing 110. For example, such a dressing interface may be a SENSAT.R.A.C.^{™} Pad available from Kinetic Concepts, Inc. of San Antonio, Texas.

The therapy system 100 may also include a regulator or controller, such as a controller 130. Additionally, the therapy system 100 may include sensors to measure operating parameters and provide feedback signals to the controller 130 indicative of the operating parameters. As illustrated in Figure 1, for example, the therapy system 100 may include a first sensor 135 and a second sensor 140 coupled to the controller 130.

The therapy system 100 may also include a source of instillation solution. For example, a solution source 145 may be fluidly coupled to the dressing 110, as illustrated in the example embodiment of Figure 1. The solution source 145 may be fluidly coupled to a positive-pressure source such as a positive-pressure source 150, a negative-pressure source such as the negative-pressure source 105, or both in some embodiments. A regulator, such as an instillation regulator 155, may also be fluidly coupled to the solution source 145 and the dressing 110 to ensure proper dosage of instillation solution (e.g. saline) to a tissue site. For example, the instillation regulator 155 may comprise a piston that can be pneumatically actuated by the negative-pressure source 105 to draw instillation solution from the solution source during a negative-pressure interval and to instill the solution to a dressing during a venting interval. Additionally or alternatively, the controller 130 may be coupled to the negative-pressure source 105, the positive-pressure source 150, or both, to control dosage of instillation solution to a tissue site. In some embodiments, the instillation regulator 155 may also be fluidly coupled to the negative-pressure source 105 through the dressing 110, as illustrated in the example of Figure 1.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 105 may be combined with the controller 130, the solution source 145, and other components into a therapy unit.

In general, components of the therapy system 100 may be coupled directly or indirectly. For example, the negative-pressure source 105 may be directly coupled to the container 115 and may be indirectly coupled to the dressing 110 through the container 115. Coupling may include fluid, mechanical, thermal, electrical, or chemical coupling (such as a chemical bond), or some combination of coupling in some contexts. For example, the negative-pressure source 105 may be electrically coupled to the controller 130 and may be fluidly coupled to one or more distribution components to provide a fluid path to a tissue site. In some embodiments, components may also be coupled by virtue of physical proximity, being integral to a single structure, or being formed from the same piece of material.

A negative-pressure supply, such as the negative-pressure source 105, may be a reservoir of air at a negative pressure or may be a manual or electrically-powered device, such as a vacuum pump, a suction pump, a wall suction port available at many healthcare facilities, or a micro-pump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure provided by the negative-pressure source 105 may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between - 50 mm Hg (-6.7 kPa) and -300 mm Hg (-39.9 kPa).

The container 115 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A controller, such as the controller 130, may be a microprocessor or computer programmed to operate one or more components of the therapy system 100, such as the negative-pressure source 105. In some embodiments, for example, the controller 130 may be a microcontroller, which generally comprises an integrated circuit containing a processor core and a memory programmed to directly or indirectly control one or more operating parameters of the therapy system 100. Operating parameters may include the power applied to the negative-pressure source 105, the pressure generated by the negative-pressure source 105, or the pressure distributed to the tissue interface 120, for example. The controller 130 is also preferably configured to receive one or more input signals, such as a feedback signal, and programmed to modify one or more operating parameters based on the input signals.

Sensors, such as the first sensor 135 and the second sensor 140, are generally known in the art as any apparatus operable to detect or measure a physical phenomenon or property, and generally provide a signal indicative of the phenomenon or property that is detected or measured. For example, the first sensor 135 and the second sensor 140 may be configured to measure one or more operating parameters of the therapy system 100. In some embodiments, the first sensor 135 may be a transducer configured to measure pressure in a pneumatic pathway and convert the measurement to a signal indicative of the pressure measured. In some embodiments, for example, the first sensor 135 may be a piezo-resistive strain gauge. The second sensor 140 may optionally measure operating parameters of the negative-pressure source 105, such as a voltage or current, in some embodiments. Preferably, the signals from the first sensor 135 and the second sensor 140 are suitable as an input signal to the controller 130, but some signal conditioning may be appropriate in some embodiments. For example, the signal may need to be filtered or amplified before it can be processed by the controller 130. Typically, the signal is an electrical signal, but may be represented in other forms, such as an optical signal.

The tissue interface 120 can be generally adapted to partially or fully contact a tissue site. The tissue interface 120 may take many forms, and may have many sizes, shapes, or thicknesses, depending on a variety of factors, such as the type of treatment being implemented or the nature and size of a tissue site. For example, the size and shape of the tissue interface 120 may be adapted to the contours of deep and irregular shaped tissue sites. Any or all of the surfaces of the tissue interface 120 may have an uneven, coarse, or jagged profile.

In some embodiments, the tissue interface 120 may comprise or consist essentially of a manifold. A manifold in this context may comprise or consist essentially of a means for collecting or distributing fluid across the tissue interface 120 under pressure. For example, a manifold may be adapted to receive negative pressure from a source and distribute negative pressure through multiple apertures across the tissue interface 120, which may have the effect of collecting fluid from across a tissue site and drawing the fluid toward the source. In some embodiments, the fluid path may be reversed or a secondary fluid path may be provided to facilitate delivering fluid, such as fluid from a source of instillation solution, across a tissue site.

In some illustrative embodiments, a manifold may comprise a plurality of pathways, which can be interconnected to improve distribution or collection of fluids. In some illustrative embodiments, a manifold may comprise or consist essentially of a porous material having interconnected fluid pathways. Examples of suitable porous material that can be adapted to form interconnected fluid pathways (e.g., channels) may include cellular foam, including open-cell foam such as reticulated foam; porous tissue collections; and other porous material such as gauze or felted mat that generally include pores, edges, and/or walls. Liquids, gels, and other foams may also include or be cured to include apertures and fluid pathways. In some embodiments, a manifold may additionally or alternatively comprise projections that form interconnected fluid pathways. For example, a manifold may be molded to provide surface projections that define interconnected fluid pathways.

In some embodiments, the tissue interface 120 may comprise or consist essentially of reticulated foam having pore sizes and free volume that may vary according to needs of a prescribed therapy. For example, reticulated foam having a free volume of at least 90% may be suitable for many therapy applications, and foam having an average pore size in a range of 400-600 microns (40-50 pores per inch) may be particularly suitable for some types of therapy. The tensile strength of the tissue interface 120 may also vary according to needs of a prescribed therapy. For example, the tensile strength of foam may be increased for instillation of topical treatment solutions. The 25% compression load deflection of the tissue interface 120 may be at least 0.35 pounds per square inch, and the 65% compression load deflection may be at least 0.43 pounds per square inch. In some embodiments, the tensile strength of the tissue interface 120 may be at least 10 pounds per square inch. The tissue interface 120 may have a tear strength of at least 2.5 pounds per inch. In some embodiments, the tissue interface may be foam comprised of polyols such as polyester or polyether, isocyanate such as toluene diisocyanate, and polymerization modifiers such as amines and tin compounds. In some examples, the tissue interface 120 may be reticulated polyurethane foam such as found in GRANUFOAM^{™} dressing or V.A.C. VERAFLO^{™} dressing, both available from Kinetic Concepts, Inc. of San Antonio, Texas.

The thickness of the tissue interface 120 may also vary according to needs of a prescribed therapy. For example, the thickness of the tissue interface may be decreased to reduce tension on peripheral tissue. The thickness of the tissue interface 120 can also affect the conformability of the tissue interface 120. In some embodiments, a thickness in a range of about 5 millimeters to 10 millimeters may be suitable.

The tissue interface 120 may be either hydrophobic or hydrophilic. In an example in which the tissue interface 120 may be hydrophilic, the tissue interface 120 may also wick fluid away from a tissue site, while continuing to distribute negative pressure to the tissue site. The wicking properties of the tissue interface 120 may draw fluid away from a tissue site by capillary flow or other wicking mechanisms. An example of a hydrophilic material that may be suitable is a polyvinyl alcohol, open-cell foam such as V.A.C. WHITEFOAM^{™} dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

In some embodiments, the tissue interface 120 may be constructed from bioresorbable materials. Suitable bioresorbable materials may include, without limitation, a polymeric blend of polylactic acid (PLA) and polyglycolic acid (PGA). The polymeric blend may also include, without limitation, polycarbonates, polyfumarates, and capralactones. The tissue interface 120 may further serve as a scaffold for new cell-growth, or a scaffold material may be used in conjunction with the tissue interface 120 to promote cell-growth. A scaffold is generally a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials.

In some embodiments, the cover 125 may provide a bacterial barrier and protection from physical trauma. The cover 125 may also be constructed from a material that can reduce evaporative losses and provide a fluid seal between two components or two environments, such as between a therapeutic environment and a local external environment. The cover 125 may comprise or consist of, for example, an elastomeric film or membrane that can provide a seal adequate to maintain a negative pressure at a tissue site for a given negative-pressure source. The cover 125 may have a high moisture-vapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, an MVTR up to 5,000 grams per square meter per twenty-four hours may provide effective breathability and mechanical properties.

In some example embodiments, the cover 125 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained. The cover 125 may comprise, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polymide copolymers. Such materials are commercially available as, for example, Tegaderm^{®} drape, commercially available from 3M Company, Minneapolis Minnesota; polyurethane (PU) drape, commercially available from Avery Dennison Corporation, Pasadena, California; polyether block polyamide copolymer (PEBAX), for example, from Arkema S.A., Colombes, France; and Inspire 2301 and Inpsire 2327 polyurethane films, commercially available from Expopack Advanced Coatings, Wrexham, United Kingdom. In some embodiments, the cover 125 may comprise INSPIRE 2301 having an MVTR (upright cup technique) of 2600 g/m²/24 hours and a thickness of about 30 microns.

An attachment device may be used to attach the cover 125 to an attachment surface, such as undamaged epidermis, a gasket, or another cover. The attachment device may take many forms. For example, an attachment device may be a medically-acceptable, pressure-sensitive adhesive configured to bond the cover 125 to epidermis around a tissue site. In some embodiments, for example, some or all of the cover 125 may be coated with an adhesive, such as an acrylic adhesive, which may have a coating weight of about 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

The solution source 145 may also be representative of a container, canister, pouch, bag, or other storage component, which can provide a solution for instillation therapy. Compositions of solutions may vary according to a prescribed therapy, but examples of solutions that may be suitable for some prescriptions include hypochlorite-based solutions, silver nitrate (0.5%), sulfur-based solutions, biguanides, cationic solutions, and isotonic solutions.

The fluid mechanics of using a negative-pressure source to reduce pressure in another component or location, such as within a sealed therapeutic environment, can be mathematically complex. However, the basic principles of fluid mechanics applicable to negative-pressure therapy and instillation are generally well-known to those skilled in the art, and the process of reducing pressure may be described illustratively herein as "delivering," "distributing," or "generating" negative pressure, for example.

In general, exudate and other fluid flow toward lower pressure along a fluid path. Thus, the term "downstream" typically implies a position in a fluid path relatively closer to a source of negative pressure or further away from a source of positive pressure. Conversely, the term "upstream" implies a position relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications, such as by substituting a positive-pressure source for a negative-pressure source, and this descriptive convention should not be construed as a limiting convention.

Negative pressure applied across the tissue site through the tissue interface 120 in the sealed therapeutic environment can induce macro-strain and micro-strain in the tissue site. Additionally, negative pressure supplied to the dressing interface 160 can induce macro-strain at the tissue site. Negative pressure can also remove exudate and other fluid from a tissue site, which can be collected in container 115.

In some embodiments, the controller 130 may receive and process data from one or more sensors, such as the first sensor 135. The controller 130 may also control the operation of one or more components of the therapy system 100 to manage the pressure delivered to the tissue interface 120. In some embodiments, controller 130 may include an input for receiving a desired target pressure and may be programmed for processing data relating to the setting and inputting of the target pressure to be applied to the tissue interface 120. In some example embodiments, the target pressure may be a fixed pressure value set by an operator as the target negative pressure desired for therapy at a tissue site and then provided as input to the controller 130. The target pressure may vary from tissue site to tissue site based on the type of tissue forming a tissue site, the type of injury or wound (if any), the medical condition of the patient, and the preference of the attending physician. After selecting a desired target pressure, the controller 130 can operate the negative-pressure source 105 in one or more control modes based on the target pressure and may receive feedback from one or more sensors to maintain the target pressure at the tissue interface 120.

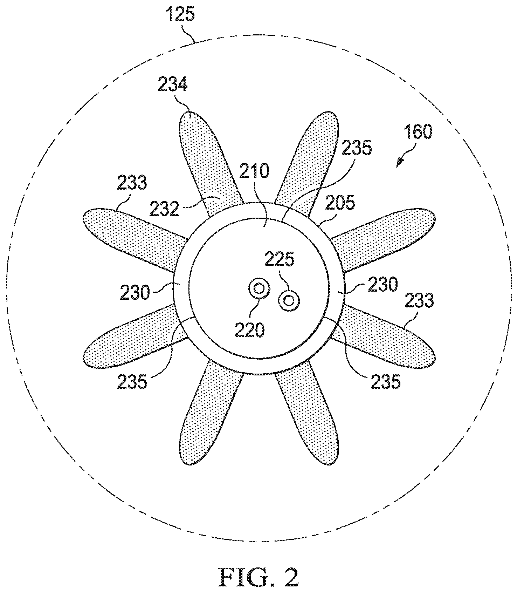

Figure 2 is a top view of the dressing interface 160, illustrating additional details that may be associated with some embodiments. The dressing interface 160 can include a base 205 having a first side 210 and a side opposite the first side 210. In some embodiments, the base 205 may comprise a circular shape. In other embodiments, the base 205 may comprise a rectangular or polygonal shape.

In some embodiments, the dressing interface 160 includes at least one supply port coupled to the base 205. For example, a first supply port 220 and a second supply port 225 may be coupled to the base 205. The first supply port 220 may be disposed proximate a center portion of the base 205 and extend through the base 205 from the first side 210. The second supply port 225 may also be disposed in the base 205 proximate the first supply port 220 and extend through the base 205 from the first side 210. In other embodiments, the base 205 may only include the first supply port 220. Both the first supply port 220 and the second supply port 225 may be configured to provide a fluid coupling to the dressing interface 160. In some embodiments, the first supply port 220 and the second supply port 225 can be fluidly coupled to a fluid conductor, such as a conduit or tube. In other embodiments, the first supply port 220 and the second supply port 225 can be directly coupled to another device such as a source of negative pressure or a source of instillation fluid.

The dressing interface 160 may also include a flap 230. The flap 230 can be coupled to a perimeter of the base 205. For example, the perimeter of the base 205 may coincide with a circumference of the base 205, and the flap 230 may be coupled to the base 205 around the circumference of the base 205. In some embodiments, the flap 230 may surround the base 205. For example, the flap 230 may be an annular body having an inner diameter substantially equal to an outer diameter of the base 205. The inner diameter of the flap 230 may be disposed adjacent to the perimeter of the base 205 so that the flap 230 surrounds the base 205. In some embodiments, the flap 230 may be formed by injection molding and comprise a polyethylene or a polypropylene material. In other embodiments, the flap 230 may comprise one or more of polyvinyl chloride, polyvinyl chloride, and polycarbonate.

In some embodiments, the flap 230 may be configured to move relative to the base 205. For example, the inner diameter of the flap 230 may be operably coupled to the perimeter of the base 205 so that the outer diameter of the flap 230 may pivot relative to the base 205 about the inner diameter of the flap 230. In some embodiments, the flap 230 may be operably coupled to the base 205 by a bearing 235. For example, the bearing 235 may couple the perimeter of the base 205 to the inner diameter of the flap 230. In some embodiments, the bearing 235 can be a flexure bearing, integral hinge, or a living hinge. For example, the base 205 and the flap 230 can comprise integral components formed from a body of the same or similar material. The body having the base 205 and the flap 230 can be thinned so that a thickness of the body is reduced at the at the inner diameter of the flap 230. The thinned portion comprising the bearing 235 can separate the base 205 from the flap 230. The bearing 235 can allow an outer diameter of the flap 230 to move relative to the base 205. For example, the dressing interface 160 can be configured to form a space sealed from the ambient environment. Negative pressure can be supplied to that space, and under a supply of negative pressure to one or both of the first supply port 220 and the second supply port 225, the bearing 235 may permit the outer diameter of the flap 230 to rotate inward toward the center of the base 205 about the bearing 235.

The dressing interface 160 may also include a plurality of force distribution components or strips 233. The plurality of strips 233 may be circumferentially disposed about the base 205. Each strip 233 may comprise an elongated body having a first end 232 and a second end 234. In some embodiments, each strip 233 may have a generally rectangular shape having a rounded ovular end. For example, the first end 232 of each strip 233 may be substantially rectangular and be coupled to the flap 230. The strip 233 may extend from the first end 232 radially away from the flap 230 to the second end 234. In some embodiments, the plurality of strips 233 may comprise a polyurethane material. In some additional embodiments, the plurality of strips 233 may comprise one or more of a polystyrene, nylon, linear low-density polyethylene, high density polyethylene, and polypropylene.

In some embodiments, each of the plurality of strips 233 may also be configured to be coupled to the cover 125. For example, the second end 234 of each of the strips 233 may be coupled to the cover 125. In some embodiments, the second end 234 of each of the strips 233 may be coupled adjacent peripheral edges of the cover 125. In other embodiments, a length of each strip 233 between the first end 232 and the second end 234 may be coupled to the cover 125. Coupling between the strip 233 and the cover 125 can be by methods suitable for use in medical applications. For example, the strip 233 may be coupled to the cover 125 by an adhesive similar to the attachment device of the cover 125. In other embodiments, coupling between the plurality of strips 233 and the cover 125 may be preassembled.

In some embodiments, rotation of the flap 230 about the bearing 235 may cause each of the strips 233 to move relative to the base 205. For example, as the flap 230 rotates toward the base 205, the movement of the flap 230 may draw each of the strips 233 radially inward toward a center of the base 205. The radially inward motion of the plurality of strips 233 may also be translated to the cover 125 through the coupling of the plurality of strips 233 to the cover 125. The movement of the strips 233 can develop a force drawing edges of the cover 125 radially inward.

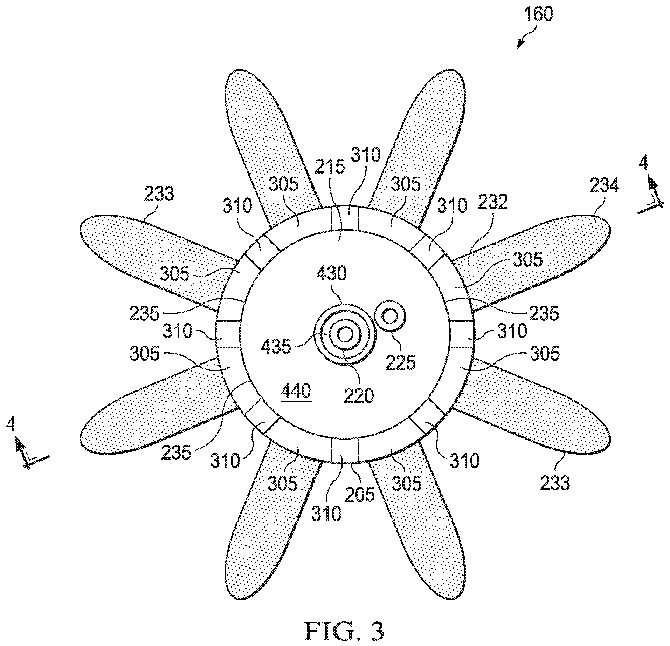

Figure 3 is a bottom view of the dressing interface 160, illustrating additional details that may be associated with some embodiments. As illustrated in Figure 3, the side of the base 205 opposite the first side 210 is a second side 215. The second side 215 may be configured to be positioned adjacent a tissue site. For example, the second side 215 of the base 205 may be configured to face to the cover 125.

In some embodiments, the flap 230 can comprise a plurality of discrete segments 305 connected to adjacent sections by a plurality of joints 310. For example, during formation of the dressing interface 160, the base 205 and the flap 230 can be formed by creating the bearing 235 identifying the inner diameter of the flap 230 and a perimeter of the base 205. Portions of the annular body comprising the flap 230 can be removed to create the segments 305, allowing the segments 305 to move relative to the base 205 independently of each other. In some embodiments, a strip 233 may be coupled to each segment 305. In other embodiments, the number of strips 233 may not be equal to the number of segments 305.

In some embodiments, each of the joints 310 can couple adjacent segments 305 to each other. For example, the joints 310 can comprise over-molded components formed from a material permitting the adjacent segments 305 to maintain a degree of freedom of movement while being able to maintain a fluid seal between the segments 305. In some embodiments, the joints 310 can comprise collapsible features configured to at least partially collapse, allowing the segments 305 to rotate radially inward, drawing the plurality of strips 233 coupled to the segments 305 radially inward. In some embodiments, the collapsible features may comprise liquid silicon rubbers, thermoplastic polyurethanes, thermoplastic vulcanizates, or thermoplastic copolyester elastomers. In other embodiments, the collapsible features comprise a foam. Additionally or alternatively, the joints 310 allow for a fluid seal to be formed between the outer perimeter of the base 205, the flap 230, and the tissue site.

In some embodiments, the dressing interface 160 includes a support 430. The support 430 can comprise an annular wall coupled to the second side 215 of the base 205. In some embodiments, the support 430 can surround the first supply port 220, creating a negative-pressure chamber 435 disposed inboard of the support 430 and an annular chamber 440 disposed outboard of the support 430. The support 430 may have a height extending from the second side 215 of the base 205. In some embodiments, the height of the support 430 may be greater than a depth of the flap 230. In some embodiments, the negative-pressure chamber 435 is fluidly coupled to the first supply port 220, and the annular chamber 440 is fluidly coupled to the second supply port 225.

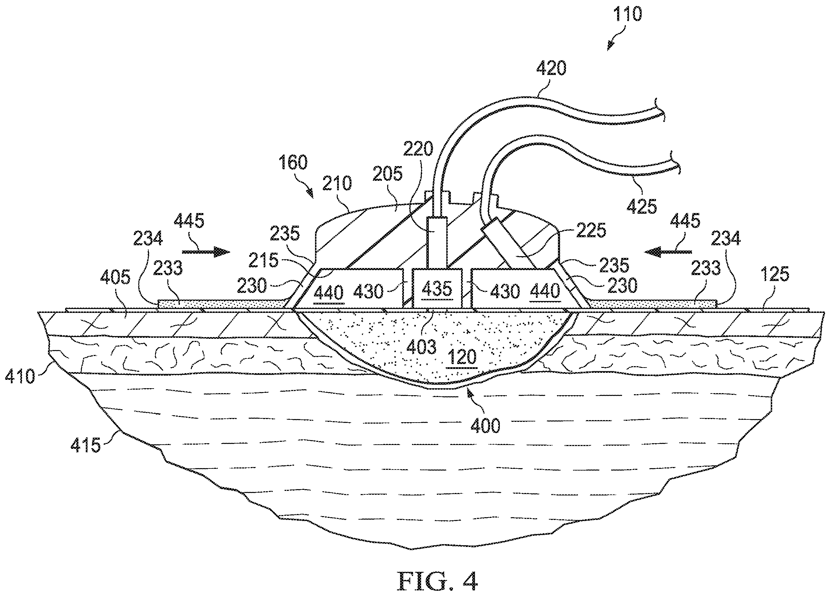

Figure 4 is a cross-section view of the dressing interface 160 taken along line 4-4 of Figure 3, illustrating additional details that may be associated with some embodiments. In operation, the tissue interface 120 may be placed within, over, on, or otherwise proximate to a tissue site, such as a tissue site 400. If the tissue site 400 is a wound, for example, the tissue interface 120 may partially or completely fill the wound, or it may be placed over the wound. In the example embodiment of Figure 4, the tissue site 400 extends through an epidermis 405, or generally skin, and a dermis 410 reaching into a hypodermis, or a subcutaneous tissue 415.

The cover 125 may be placed over the tissue interface 120 and sealed to an attachment surface near the tissue site 400. For example, the cover 125 may be sealed to undamaged epidermis peripheral to the tissue site 400. Thus, the dressing 110 can provide a sealed therapeutic environment proximate to the tissue site 400, substantially isolated from the external environment. The dressing interface 160 may be coupled to the cover 125. For example, the second end 234 of each of the plurality of strips 233 may be coupled to the cover 125. In some embodiments, the second end 234 of each of the plurality of strips 233 may be coupled to a periwound area of the tissue site 400. In other embodiments, the second end 234 of each of the plurality of strips 233 may be coupled to an edge of the tissue site 400. In still other embodiments, the second end 234 of the each of the plurality of strips 233 may be coupled to the cover 125 within an edge of the tissue site 400.

If the dressing interface 160 is coupled to the cover 125, the height of the support 430 may permit the support 430 to contact the cover 125. In some embodiments, the cover 125 may include an opening or aperture 403 extending through the cover 125. The support 430 may surround the aperture 403, fluidly coupling the negative-pressure chamber 435 to the tissue site 400 through an aperture 403. In some embodiments, the negative-pressure chamber 435 may be configured to fluidly couple the negative-pressure source 105 to the tissue site 400 via the first supply port 220. For example, the first supply port 220 may be fluidly coupled to the negative-pressure source 105 by a tube or conduit, such as a first conduit 420, and fluidly coupled to the tissue site 400 through the aperture 403 in the cover 125.

In some embodiments, the annular chamber 440 may circumscribe the negative-pressure chamber 435 and be fluidly isolated from the negative-pressure chamber 435 by the support 430 and the cover 125. As shown in Figure 4, the second supply port 225 may extend from the first side 210 toward an outer perimeter of the second side 215 of the base 205 to be fluidly coupled to the annular chamber 440. The second supply port 225 may be configured to fluidly couple a negative pressure source, such as the negative-pressure source 105, to the annular chamber 440 by a tube or conduit, such as a second conduit 425. In other embodiments, the second conduit 425 may fluidly couple the second supply port 225 to another negative pressure source separate from the negative-pressure source 105.

In some embodiments, the first supply port 220 is configured to receive a first pressure and the second supply port 225 is configured to receive a second pressure. The first pressure may be different from the second pressure. For example, the second pressure may be lower than the first pressure. In other embodiments, the first pressure and the second pressure may be equal. In still other embodiments, the second pressure may be higher than the first pressure. In some embodiments, the negative-pressure source 105 may be fluidly coupled to the first supply port 220 and the second supply port 225 to supply the first pressure and the second pressure. In other embodiments, the negative-pressure source 105 may be a first negative pressure source configured to be fluidly coupled to the first supply port 220 and supply the first pressure. In such embodiments, a second negative pressure source may be configured to be coupled to the second supply port 225 and supply the second pressure.

The first pressure may be supplied through the first supply port 220 of the dressing interface 160 and delivered to the tissue site 400 through the aperture 403 in the cover 125. Additionally or alternatively, a second pressure may be supplied through the second supply port 225 of dressing interface 160. The second pressure supplied through the second supply port 225 may cause the flap 230 to move rotate radially inward about the bearing 235, pulling the strips 233 radially inward toward a center of the base 205, as indicated by the arrows 445. The force generated by the inward motion of the flap 230 and strips 233 may be translated through the cover 125 to a periwound area of the tissue site 400 to draw edges of the tissue site 400 inward. The force may also be configured to hold tension at the tissue site 400.

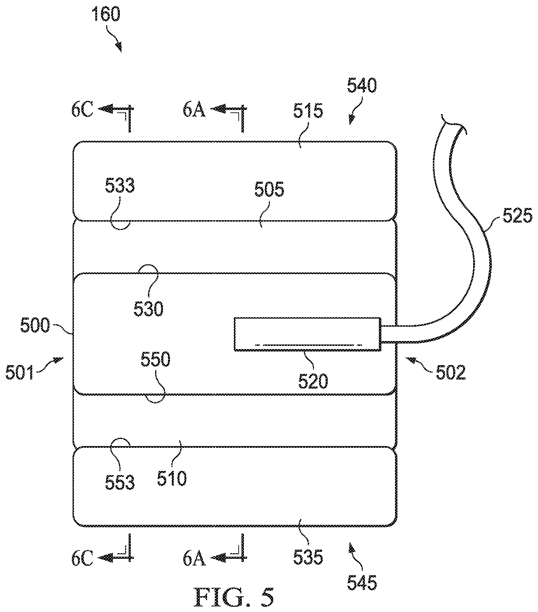

Figure 5 is a top view of another embodiment of the dressing interface 160, illustrating additional details that may be associated with some embodiments. In some embodiments, the dressing interface 160 includes a bolster body 500, a first flange 505, a second flange 510, a first tab 515, and a second tab 535. The bolster body 500 may be configured to be fluidly coupled to a source of negative pressure, such as the negative-pressure source 105. For example, the bolster body 500 may include a negative-pressure interface 520. The negative-pressure interface 520 may fluidly couple the negative-pressure source 105 to the dressing interface 160 through a tube or conduit, such as a conduit 525. In some embodiments, one or more of the bolster body 500, the first flange 505, the second flange 510, the first tab 515, and the second tab 535 may comprise a polyethylene, a polypropylene, a polyvinyl chloride, or an acrylonitrile butadiene styrene material.

In some embodiments, the bolster body 500 includes a first end 501 and a second end 502. The first end 501 and the second end 502 may each include a wall that define a chamber of the bolster body 500 with at least the first flange 505 and the second flange 510. In other embodiments, at least one sealing layer may be positioned over the first end 501 and the second end 502 to maintain a fluid seal within the chamber of the bolster body 500. For example, the at least one sealing layer may comprise a film configured to be coupled to the first end 501 and the second end 502 and at least a portion of the cover 125 or tissue surrounding the tissue site with an adhesive.

In some embodiments, the first flange 505 may be coupled to a first longitudinal side 540 of the bolster body 500, and the second flange 510 may be coupled to a second longitudinal side 545 of the bolster body 500. The second longitudinal side 545 may be opposite the first longitudinal side 540. In some embodiments, the first flange 505 may be operably coupled to the bolster body 500 so that the first flange 505 may move relative to the bolster body 500. For example, an edge of the first flange 505 proximal to the bolster body 500 may be affixed to the bolster body 500. The first flange 505 may extend from the bolster body 500 to another edge distal from the bolster body 500. The distal edge of the first flange 505 may rotate relative to the bolster body by the proximal edge of the first flange 505. In some embodiments, the proximal edge of the first flange 505 may be a first bearing 530. In some embodiments, a supply of negative pressure to the bolster body 500 may cause the first flange 505 to rotate relative to the bolster body about the first bearing 530.

In some embodiments, the second flange 510 may be operably coupled to the bolster body 500 so that the second flange 510 may move relative to the bolster body 500. For example, an edge of the second flange 510 proximal to the bolster body 500 may be affixed to the bolster body 500. The second flange 510 may extend from the bolster body 500 to another edge distal from the bolster body 500. The distal edge of the second flange 510 may rotate relative to the bolster body 500 by the proximal edge of the second flange 510. In some embodiments, the proximal edge of the second flange 510 may be a second bearing 550. In some embodiments, a supply of negative pressure to the bolster body 500 may cause the second flange 510 to rotate relative to the bolster body 500 about the second bearing 550.

Each of the first bearing 530 and the second bearing 550 can be a flexure bearing or a living hinge. For example, the first flange 505, the second flange 510, and the bolster body 500 can comprise integral components formed from a body of the same or similar material. The body can be thinned so that a thickness of the body is reduced at the at the first bearing 530 and the second bearing 550. The thinned portion comprising the first bearing 530 and the second bearing 550 can separate the bolster body 500 from the first flange 505 and the second flange 510, respectively. The first bearing 530 and the second bearing 550 can allow the distal edges of the first flange 505 and the second flange 510 to move relative to the bolster body 500. For example, the dressing interface 160 can be configured to form a space sealed from the ambient environment. Negative pressure can be supplied to that space, and under a supply of negative pressure to negative-pressure interface 520, the first bearing 530 and the second bearing 550 may permit the distal edges of the first flange 505 and the second flange 510 to rotate inward toward a center of the bolster body 500 about the first bearing 530 and the second bearing 550.

In some embodiments, the first tab 515 can be coupled to the distal edge of the first flange 505, and the second tab 535 can be coupled to the distal edge of the second flange 510. The first tab 515 and the second tab 535 can be configured to couple the first flange 505 and the second flange 510 to the cover 125. In some embodiments, the first tab 515 and the second tab 535 can comprise substantially flat polygonal bodies having a first edge coupled to the distal edge of the first flange 505 and the second flange 510, respectively. Additionally or alternatively, the distal edge of the first flange and the second flange 510 may be a third bearing 533 and a fourth bearing 553, respectively. The third bearing 533 and the fourth bearing may be similar or analogous to the first bearing 530 and the second bearing 550. In some embodiments, the first tab 515 and the second tab 535 may have a surface having an adhesive coating or layer disposed on the surface and configured to adhere to the cover 125.

In some embodiments, the bolster body 500, the first flange 505, the second flange 510, the first tab 515, and the second tab 535 may each comprise a rectangular shape. In other embodiments, one or more of the bolster body 500, the first flange 505, the second flange 510, the first tab 515 and the second tab 535 may comprise an ovular shape. In still other embodiments, one or more of the bolster body 500, the first flange 505, the second flange 510, the first tab 515, and the second tab 535 may comprise a polygonal shape.

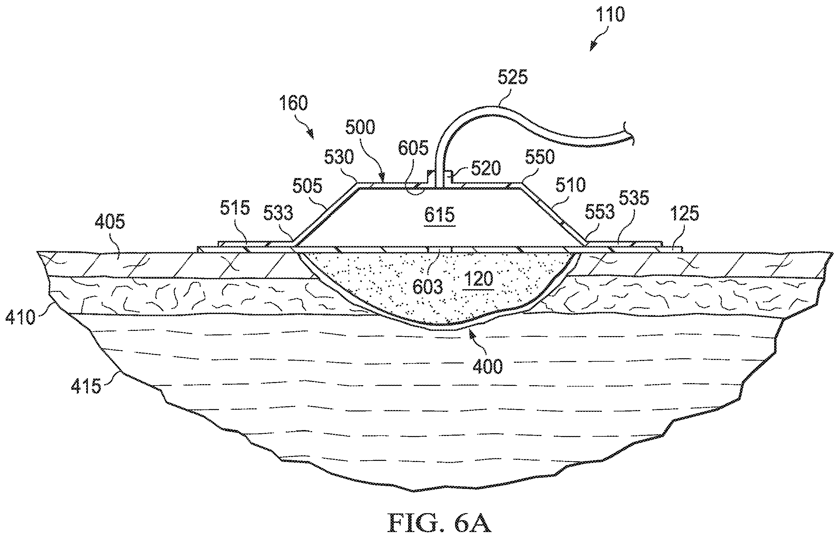

Figure 6A is a cross-section of the dressing interface 160 through line 6A-6A of Figure 5, illustrating additional details that may be associated with some embodiments. In some embodiments, the first tab 515 and the second tab 535 can be adhered to the cover 125 to couple the dressing interface 160 to the cover 125. If the first tab 515 and the second tab 535 are coupled to the cover 125, the bolster body 500 forms a chamber 615 between the bolster body 500 and the cover 125. In some embodiments, the chamber 615 can be fluidly coupled to the negative-pressure source 105 via the negative-pressure interface 520 and the conduit 525. In some embodiments, the chamber 615 may be configured to be fluidly coupled to the tissue site 400 through an aperture 603 in the cover 125. In other embodiments, the chamber 615 may be fluidly isolated from the tissue site by the cover 125.

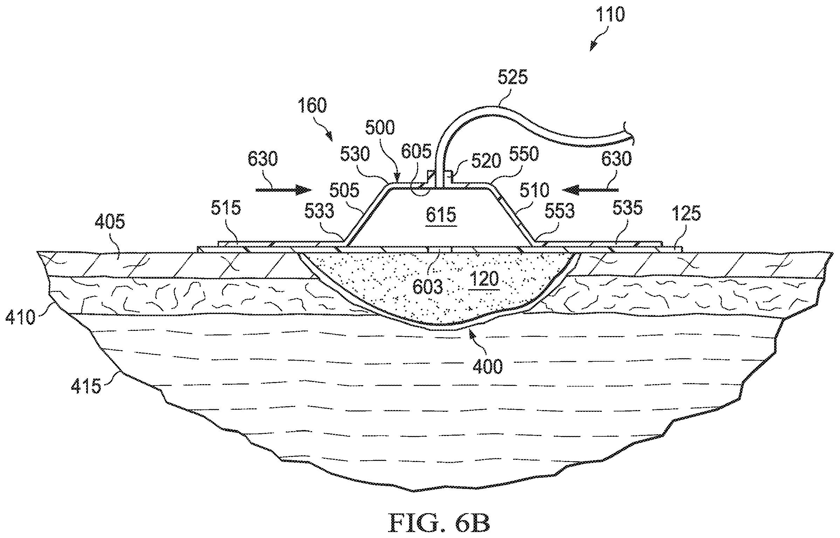

Figure 6B is a cross-section of the dressing interface 160 through line 6A-6A of Figure 5, illustrating additional details that may be associated with operation of the dressing interface 160. With reference to Figure 6B, the first flange 505 and the second flange 510 may be configured to develop a closing force parallel to the tissue site 400, indicated by arrows 630, in response to a supply of negative pressure to the chamber 615. For example, negative pressure may be supplied from the negative-pressure source 105 to the chamber 615 via the conduit 525 and the negative-pressure interface 520. In response, negative pressure may be delivered to the tissue site 400 via the aperture 603 in the cover 125. In some embodiments, the negative pressure developed in the chamber 615 can urge the first flange 505 and the second flange 510 to rotate about the first bearing 530 and the second bearing 550. For example, the differential pressure between the chamber 615 and the ambient environment across the first flange 505 and the second flange 510 can urge the first flange 505 and the second flange 510 toward a center of the chamber 615. As the proximal edges of the first flange 505 and the second flange 510 are affixed to the bolster body 500, the distal edges of the first flange 505 and the second flange 510 will rotate inward toward the center of the chamber 615. For example, the distal edges of the first flange 505 and the second flange 510 may rotate about the third bearing 533 and the fourth bearing 553. In response, the first tab 515 and the second tab 535 may move horizontally toward the chamber 615 through the coupling between the first flange 505 and the first tab 515 and the second flange 510 and the second tab 535. The movement of the first tab 515 and the second tab 535 through the coupling to the cover 125 generates a force toward a center of the chamber 615 that urges edges of the tissue site 400 to close, a closing force in the direction of the arrows 630. In some additional embodiments, mechanical fasteners may be applied to hold tension at the tissue site 400 after negative pressure has been supplied to the bolster body 500.

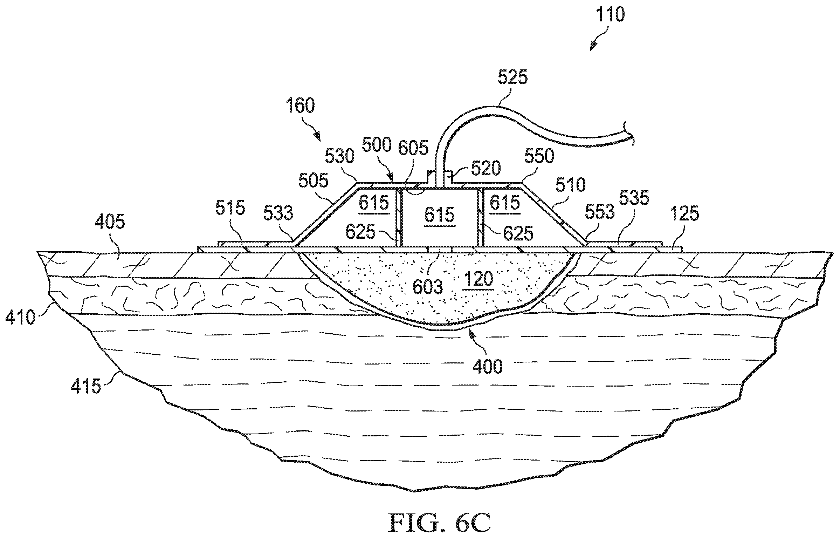

Figure 6C is a cross-section of the dressing interface 160 through line 6C-6C of Figure 5, illustrating additional details that may be associated with some embodiments. In some embodiments, one or more support columns 625 may be disposed in the chamber 615 of the bolster body 500. Each of the support columns 625 may extend from a surface 605 of the bolster body 500 configured to face the cover 125. In some embodiments, the support columns 625 may be configured to be coupled to the cover 125, such as with an adhesive. The support columns 625 may also be spaced apart along a length and a width of the bolster body 500 within the chamber 615. Each of the support columns 625 may be comprise a rigid member configured prevent collapse of chamber 615 of the bolster body 500 if a negative pressure is developed in the chamber 615. For example, the support column 625 may comprise a polyethylene, a polypropylene, a polyvinyl chloride, an acrylonitrile butadiene styrene, or a polycarbonate material.

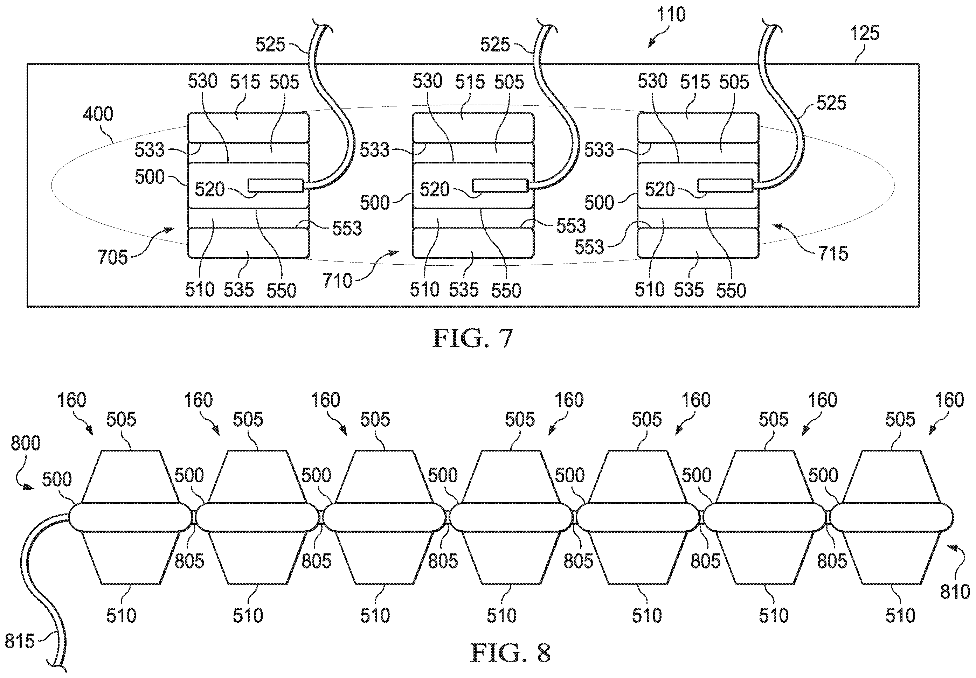

Figure 7 is a top view of another example embodiment of the dressing 110, illustrating additional details that may be associated with some embodiments. For example, the dressing 110 may include a plurality of the dressing interfaces 160, such as a first dressing interface 705, a second dressing interface 710, and a third dressing interface 715. Each of the first dressing interface 705, the second dressing interface 710, and the third dressing interface 715 may be similar or analogous to the dressing interfaces 160 discussed above with respect to Figures 5-6C.

As shown in Figure 7, the first dressing interface 705, the second dressing interface 710, and the third dressing interface 715 may be coupled to the cover 125 and positioned in series along the tissue site 400. For example, the tissue site 400 may be an incision, and the first dressing interface 705, the second dressing interface 710, and the third dressing interface 715 may be axially aligned along the tissue site 400. In other embodiments, one or more of the first dressing interface 705, the second dressing interface 710, and the third dressing interface 715 may be offset from each other. For example, the tissue site 400 may be a non-linear wound or incision and the first dressing interface 705, the second dressing interface 710, and the third dressing interface 715 may be centered over the tissue site 400 and positioned along a length of the tissue site 400.

In some embodiments, the first dressing interface 705, the second dressing interface 710, and the third dressing interface 715 may be fluidly coupled to a negative pressure source, such as the negative-pressure source 105, by the conduit 525. Under a supply of negative pressure from the negative-pressure source 105, each of the plurality of dressing interfaces 160 may developed a closing force parallel to the tissue site 400 and draw edges of the tissue site 400 toward each other. In other embodiments, the first dressing interface 705, the second dressing interface 710, and the third dressing interface 715 may be fluidly coupled to a different negative pressure source. For example, the first dressing interface 705 may be fluidly coupled to a first negative pressure source, the second dressing interface 710 may be fluidly coupled to a second negative pressure source, and the third dressing interface 715 may be fluidly coupled to a third negative pressure source. In such embodiments, the first negative pressure source, the second negative pressure source, and the third negative pressure source may be configured to supply a first pressure, a second pressure and a third pressure, respectively. One or more of the first pressure, the second pressure, and the third pressure may be different such that the first dressing interface 705, the second dressing interface 710, and the third dressing interface 715 develop a different closing force at the tissue site 400. In other embodiments, the first pressure, the second pressure and the third pressure may be equal. In still other embodiments, the first pressure, the second pressure, and the third pressure may be delivered to the respective dressing interfaces 160 sequentially.

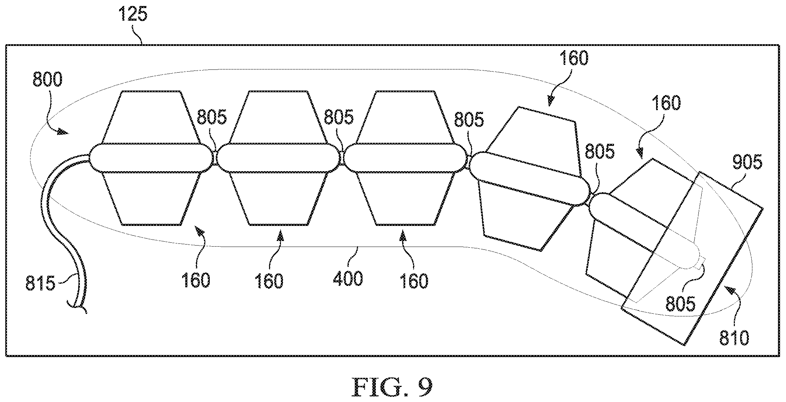

Figure 8 is a top view of another example embodiment of the dressing interface 160, illustrating additional details that may be associated with some embodiments. In some embodiments, the dressing interface 160 comprises a plurality of dressing interfaces 160. Similar to the dressing interfaces 160 of Figures 5-7, each of the dressing interfaces 160 may comprise the bolster body 500, the first flange 505, and the second flange 510. In some embodiments, the bolster body 500 may comprise an elongated body having ovular ends. In other embodiments, the bolster body 500 may comprise a rectangular, round, or polygonal shape. In some embodiments, the first flange 505 and the second flange 510 comprise a polygonal shape. In other embodiments, the first flange 505 and the second flange 510 comprise a rectangular, ovular, or round shape. As illustrated in Figure 8, each of the first flange 505 and the second flange 510 of the dressing interfaces 160 may have a trapezoidal shape. The proximal edge of the first flange 505 and the second flange 510 may have a first length, and the distal edge of the first flange 505 and the second flange 510 may have a second length. In some embodiments, the second length is less than the first length. In such embodiments, the shape of the first flange 505 and the second flange 510 allows each bolster body 500 to bend into a non-linear configuration, as shown in Figure 9.

Each of the plurality of dressing interfaces 160 may be connected by flexion or articulation joints 805 to form a series of the dressing interfaces 160. The series of dressing interfaces 160 includes a first end 800 and a second end 810. The first end 800 of the series of dressing interfaces 160 may be coupled to a conduit 815. For example, the conduit may be fluidly coupled to the bolster body 500 on the first end 800. The conduit 815 may be configured to fluidly couple the negative-pressure source 105 to the series of dressing interfaces 160.

The articulation joints 805 are configured to fluidly couple each bolster body 500 of the series of dressing interfaces 160 to each other and to the negative-pressure source 105. For example, the articulation joints 805 may comprise a tube or conduit fluidly coupling each bolster body 500 to an adjacent bolster body. The articulation joints 805 are also configured to fluidly couple each of the bolster bodies 500 to the negative-pressure source 105 via the conduit 815. The negative-pressure source 105 may be configured to deliver reduced pressure to each of the dressing interfaces 160 in the series such that each of the dressing interfaces 160 develops a closing force drawings edges of the tissue site toward each other.

Figure 9 is a top view of the series of dressing interfaces 160 of Figure 8, illustrating an operative example according to some embodiments. The series of the dressing interfaces 160 may be configured to be coupled to the cover 125 positioned at the tissue site 400. In some embodiments, the series of the dressing interfaces 160 may be cut to achieve a desired length of the series of dressing interfaces 160. For example, one or more of the dressing interfaces 160 adjacent the second end 810 may be cut and separated at one of the articulation joints 805. The desired length may correspond to a length of the tissue site 400. Additionally, a sealing layer 905 may be positioned over the articulation joint 805 that has been cut at the second end 810 to maintain a fluid seal within the series of dressing interfaces 160. For example, the sealing layer 905 may comprise a film layer configured to be coupled to the articulation joint 805 that has been cut and at least a portion of the cover 125 with an adhesive. In some embodiments, the sealing layer 905 may comprise the same material as the cover 125.

In some embodiments, the series of dressing interfaces 160 may also be configured to rotate at the articulation joint 805. For example, as shown in Figure 9, the tissue site 400 may include a non-linear wound or incision. One or more of the articulation joints 805 may be rotated such that the series of dressing interfaces 160 are centered over and positioned along a length of the tissue site 400. In some embodiments, each of the articulation joints 805 are configured to rotate up to about 30⁰. In other embodiments, each of the articulation joints 805 are configured to rotate about 90⁰ or less. In some embodiments, the first flange 505 and the second flange 510 of each dressing interface 160 can be shaped to accommodate articulation of adjacent dressing connectors so that, if coupled to the cover 125, the adjacent flaps may not interfere with each other.
The systems, apparatuses, and methods described herein may provide significant advantages. For example, the embodiments described herein may provide a dressing interface 160 that provides macrostrain to a wound at a tissue site separate from any negative pressure that may be applied to the wound itself. Higher levels of macrostrain may be achieved by applying lower pressure to the wound edge than applied to the wound itself. The dressing interface 160 may create a force at the tissue site that pulls radially on the edges of the wound and generates tension at the wound. The force created at the tissue site may reduce the surface area of the wound, which may result in faster wound healing. Additionally, some embodiments of the dressing interface 160 described herein may provide a dressing interface 160 that generates a force to close an opening through a surface of the tissue site, such as an incision. The force generated by the dressing interface 160 may draw edges of the opening toward each other. In some embodiments, a plurality of the dressing interfaces 160 may be placed in series along a length of linear or non-linear wound or incision. Drawing the edges of the opening in the tissue site together may promote faster wound healing.

## Claims

1. An apparatus for treating a tissue site, comprising:
a base (205) having a first side (210) and a second side;
a first supply port (220) coupled to the base (205) and configured to be fluidly coupled to the tissue site;
a second supply port (225) coupled the base (205), the second supply port (225) configured to be fluidly coupled to the second side of the base (205);
a plurality of flaps (230) coupled to a portion of the base (205) and configured to generate a force at the tissue site under a supply of negative pressure;
a first negative-pressure source configured to be fluidly coupled to the first supply port (220); and
a second negative-pressure source configured to be fluidly coupled to the second supply port (225);
**characterized in that** the first supply port (220) is configured to receive a first pressure and the second supply port is configured to receive a second pressure, the second pressure being different than the first pressure; and
the second pressure is lower than the first pressure.

2. The apparatus of claim 1, wherein the base (205) comprises a circular shape.

3. The apparatus of claim 2, wherein the plurality of flaps (230) are positioned circumferentially about the base (205).

4. The apparatus of claim 2, wherein the force generated by the plurality of flaps (230) is radially inward toward a center of the base (205).

5. The apparatus of claim 1, wherein the plurality of flaps (230) are configured to be coupled adjacent an edge of a wound at the tissue site.

6. The apparatus of claim 1, wherein the plurality of flaps (230) comprise a foam.

7. The apparatus of claim 1, wherein the plurality of flaps (230) comprise a polyurethane material.

8. The apparatus of claim 1, wherein the first supply port (220) is configured to extend through a center of the base (205) from the first side (210) to the second side.

9. The apparatus of claim 1, wherein the second supply port (225) is configured to extend through the base (205) from the first side (210) to the second side of the base (205), the second supply port (225) adjacent the first supply port (220).

10. The apparatus of claim 1, further comprising a plurality of collapsible features coupled to an outer perimeter of the second side of the base (205).

11. The apparatus of claim 10, wherein each of the collapsible features are positioned between one or more of the flaps (230).

12. The apparatus of claim 10, wherein the collapsible features comprise a foam.

13. The apparatus of claim 10, wherein the collapsible features are configured to collapse under the supply of negative pressure.

14. The apparatus of claim 1, wherein the plurality of flaps (230) are configured to be coupled to a drape (125) positioned over the tissue site.

15. The apparatus of claim 1, wherein the force generated is configured to hold tension at the tissue site.

## Patentansprüche

1. Eine Einrichtung zum Behandeln einer Gewebestelle, aufweisend:
eine Basis (205), die eine erste Seite (210) und eine zweite Seite aufweist;
einen ersten Versorgungsanschluss (220), der mit der Basis (205) verbunden ist und so konfiguriert ist, dass er mit der Gewebestelle in Fluidverbindung steht;
einen zweiten Versorgungsanschluss (225), der mit der Basis (205) gekoppelt ist, wobei der zweite Versorgungsanschluss (225) so konfiguriert ist, dass er mit der zweiten Seite der Basis (205) in Fluidverbindung steht;
eine Mehrzahl von Klappen (230), die mit einem Abschnitt der Basis (205) gekoppelt sind und dazu konfiguriert sind, bei Zufuhr von Unterdruck eine Kraft an der Gewebestelle zu erzeugen;
eine erste Unterdruckquelle, die so konfiguriert ist, dass sie mit dem ersten Versorgungsanschluss (220) in Fluidverbindung steht; und
eine zweite Unterdruckquelle, die so konfiguriert ist, dass sie mit dem zweiten Versorgungsanschluss (225) in Fluidverbindung steht;
**dadurch gekennzeichnet, dass** der erste Versorgungsanschluss (220) so konfiguriert ist, dass er einen ersten Druck aufnimmt, und der zweite Versorgungsanschluss so konfiguriert ist, dass er einen zweiten Druck aufnimmt, wobei sich der zweite Druck vom ersten Druck unterscheidet; und
der zweite Druck niedriger ist als der erste Druck.

2. Die Einrichtung nach Anspruch 1, wobei die Basis (205) eine kreisförmige Form aufweist.

3. Die Einrichtung nach Anspruch 2, wobei die Mehrzahl von Klappen (230) umfänglich um die Basis (205) angeordnet ist.

4. Die Einrichtung nach Anspruch 2, wobei die durch die Mehrzahl von Klappen (230) erzeugte Kraft radial nach innen in Richtung einer Mitte der Basis (205) gerichtet ist.

5. Die Einrichtung nach Anspruch 1, wobei die Mehrzahl von Klappen (230) so konfiguriert ist, dass sie benachbart zu einem Rand einer Wunde an der Gewebestelle gekoppelt wird.

6. Die Einrichtung nach Anspruch 1, wobei die Mehrzahl von Klappen (230) einen Schaumstoff aufweist.

7. Die Einrichtung nach Anspruch 1, wobei die Mehrzahl von Klappen (230) ein Polyurethanmaterial aufweist.

8. Die Einrichtung nach Anspruch 1, wobei der erste Versorgungsanschluss (220) so konfiguriert ist, dass er sich durch eine Mitte der Basis (205) von der ersten Seite (210) zu der zweiten Seite erstreckt.

9. Die Einrichtung nach Anspruch 1, wobei der zweite Versorgungsanschluss (225) so konfiguriert ist, dass er sich durch die Basis (205) von der ersten Seite (210) zu der zweiten Seite der Basis (205) erstreckt, wobei der zweite Versorgungsanschluss (225) benachbart zu dem ersten Versorgungsanschluss (220) liegt.

10. Die Einrichtung nach Anspruch 1, die ferner eine Mehrzahl von zusammenklappbaren Merkmalen aufweist, die mit einem Außenumfang der zweiten Seite der Basis (205) gekoppelt ist.

11. Die Einrichtung nach Anspruch 10, wobei jedes der zusammenklappbaren Merkmale zwischen einer oder mehreren Klappen (230) positioniert ist.

12. Die Einrichtung nach Anspruch 10, wobei die zusammenklappbaren Merkmale einen Schaumstoff aufweisen.

13. Die Einrichtung nach Anspruch 10, wobei die zusammenklappbaren Elemente so konfiguriert sind, dass sie unter der Zufuhr von Unterdruck zusammenklappen.

14. Die Einrichtung nach Anspruch 1, wobei die Mehrzahl von Klappen (230) so konfiguriert ist, dass sie mit einem über der Gewebestelle positionierten Abdecktuch (125) gekoppelt werden.

15. Die Einrichtung nach Anspruch 1, wobei die erzeugte Kraft so konfiguriert ist, dass sie die Spannung an der Gewebestelle aufrechterhält.

## Revendications

1. Appareil pour le traitement d'un site tissulaire, comprenant :
une base (205) ayant un premier côté (210) et un second côté ;
un premier orifice d'alimentation (220) accouplé à la base (205) et conçu pour être relié de manière fluidique au site tissulaire ;
un second orifice d'alimentation (225) accouplé à la base (205), le second orifice d'alimentation (225) étant conçu pour être accouplé de manière fluidique au second côté de la base (205) ;
une pluralité de rabats (230) accouplés à une partie de la base (205) et conçus pour générer une force au niveau du site tissulaire sous l'effet d'une alimentation en pression négative ;
une première source de pression négative conçue pour être accouplée de manière fluidique au premier orifice d'alimentation (220) ; et
une seconde source de pression négative conçue pour être accouplée de manière fluidique au second orifice d'alimentation (225) ;
**caractérisé en ce que** le premier orifice d'alimentation (220) est conçu pour recevoir une première pression et le second orifice d'alimentation est conçu pour recevoir une seconde pression, la seconde pression étant différente de la première pression ; et
la seconde pression est inférieure à la première pression.

2. Appareil selon la revendication 1, dans lequel la base (205) comprend une forme circulaire.

3. Appareil selon la revendication 2, dans lequel la pluralité de rabats (230) sont positionnés de manière circonférentielle autour de la base (205).

4. Appareil selon la revendication 2, dans lequel la force générée par la pluralité de rabats (230) est radialement intérieure vers un centre de la base (205).

5. Appareil selon la revendication 1, dans lequel la pluralité de rabats (230) sont conçus pour être accouplés de manière à être adjacents à un bord d'une plaie au niveau du site tissulaire.

6. Appareil selon la revendication 1, dans lequel la pluralité de rabats (230) comprennent une mousse.

7. Appareil selon la revendication 1, dans lequel la pluralité de rabats (230) comprennent un matériau de type polyuréthane.

8. Appareil selon la revendication 1, dans lequel le premier orifice d'alimentation (220) est conçu pour s'étendre à travers un centre de la base (205) depuis le premier côté (210) jusqu'au second côté.

9. Appareil selon la revendication 1, dans lequel le second orifice d'alimentation (225) est conçu pour s'étendre à travers la base (205) depuis le premier côté (210) jusqu'au second côté de la base (205), le second orifice d'alimentation (225) étant adjacent au premier orifice d'alimentation (220).

10. Appareil selon la revendication 1, comprenant en outre une pluralité d'éléments pliables accouplés à un périmètre extérieur du second côté de la base (205).

11. Appareil selon la revendication 10, dans lequel chacun des éléments pliables est positionné entre un ou plusieurs des rabats (230).

12. Appareil selon la revendication 10, dans lequel les éléments pliables comprennent une mousse.

13. Appareil selon la revendication 10, dans lequel les éléments pliables sont conçus pour se replier sous l'effet d'une alimentation en pression négative.

14. Appareil selon la revendication 1, dans lequel la pluralité de rabats (230) sont conçus pour être accouplés à un drap (125) positionné sur le site tissulaire.

15. Appareil selon la revendication 1, dans lequel la force générée est conçue pour maintenir la tension au niveau du site tissulaire.
